(19) Europäisches Patentamt
European Patent Office
Office européen des brevets

(11) EP 4 064 860 B1

(12) **EUROPEAN PATENT SPECIFICATION**

(45) Date of publication and mention
of the grant of the patent:
**01.01.2025 Bulletin 2025/01**

(21) Application number: **20811370.4**

(22) Date of filing: **27.11.2020**

(51) International Patent Classification (IPC):
*A23L 33/10* (2016.01)    *A23L 33/21* (2016.01)

(52) Cooperative Patent Classification (CPC):
**A23L 33/21; A23L 33/10;** A23V 2002/00;
A23V 2200/31; A23V 2200/3202; A23V 2200/322;
A23V 2250/28; A23V 2250/284; A23V 2250/5116;
A23V 2250/5118

(86) International application number:
**PCT/EP2020/083606**

(87) International publication number:
**WO 2021/105338 (03.06.2021 Gazette 2021/22)**

(54) **NOVEL COMPOSITION FOR REDUCING STRESS DISORDERS**

NEUE ZUSAMMENSETZUNG ZUR VERRINGERUNG VON STRESSERKRANKUNGEN

NOUVELLE COMPOSITION POUR RÉDUIRE LES TROUBLES LIÉS AU STRESS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2019 EP 19211812**

(43) Date of publication of application:
**05.10.2022 Bulletin 2022/40**

(73) Proprietor: **Société des Produits Nestlé S.A.**
**1800 Vevey (CH)**

(72) Inventors:
• **BERGONZELLI DEGONDA, Gabriela**
**1030 Bussigny (CH)**
• **LAMOTHE, Lisa**
**1003 Lausanne (CH)**
• **ROCHAT, Florence**
**1820 Montreux (CH)**
• **VAN OUDENHOVE, Lukas**
**3000 Leuven (BE)**
• **VERBEKE, Kristin**
**3020 Herent (BE)**

(74) Representative: **Chautard, Cécile**
**Société des Produits Nestlé S.A.**
**Avenue Nestlé 55**
**1800 Vevey (CH)**

(56) References cited:
US-A1- 2010 322 904    US-A1- 2012 058 968
US-A1- 2015 272 982    US-A1- 2018 214 399
US-A1- 2019 029 303    US-A1- 2019 053 523
US-B1- 6 753 019

• SCHMIDT KRISTIN ET AL: "Prebiotic intake reduces the waking cortisol response and alters emotional bias in healthy volunteers", PSYCHOPHARMACOLOGY, SPRINGER VERLAG, BERLIN, DE, vol. 232, no. 10, 3 December 2014 (2014-12-03), pages 1793 - 1801, XP035491859, ISSN: 0033-3158, [retrieved on 20141203], DOI: 10.1007/S00213-014-3810-0
• SHAWN M. TALBOTT ET AL: "Effect of Coordinated Probiotic/Prebiotic/Phytobiotic Supplementation on Microbiome Balance and Psychological Mood State in Healthy Stressed Adults", FUNCTIONAL FOODS IN HEALTH AND DISEASE, vol. 9, no. 4, 30 April 2019 (2019-04-30), pages 265, XP055683326, ISSN: 2378-7007, DOI: 10.31989/ffhd.v9i4.599
• BEATRIZ GULLÓN ET AL: "Structural features and assessment of prebiotic activity of refined arabinoxylooligosaccharides from wheat bran", JOURNAL OF FUNCTIONAL FOODS, vol. 6, 12 December 2013 (2013-12-12), NL, pages 438 - 449, XP055683368, ISSN: 1756-4646, DOI: 10.1016/j.jff.2013.11.010

- JUNYI YANG ET AL: "Impact of Dietary Fiber Fermentation from Cereal Grains on Metabolite Production by the Fecal Microbiota from Normal Weight and Obese Individuals", JOURNAL OF MEDICINAL FOOD, vol. 16, no. 9, 1 September 2013 (2013-09-01), US, pages 862 - 867, XP055683828, ISSN: 1096-620X, DOI: 10.1089/ jmf.2012.0292

**Description**

**Field of the invention**

The present invention concerns an edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals to 100, to food compositions comprising such edible ingredient for use in the attenuation of stress response and/or prevention of chronic stress and of conditions thereby associated.

**Background of the invention**

There is an increasing research linking gut health with mental wellbeing, with the gut microbiome being at the center of gut-brain communication. The gut microbiome is considered to be involved in the development of mood, anxiety and stress-related disorders. The microbiome can be modulated by means of dietary interventions including probiotics and prebiotic fibers. Short chain fatty acids (SCFAs), the microbial metabolites that constitute the major products of bacterial fermentation of dietary fiber in the gut, are often considered key candidate mediators of the impact of the microbiome on the brain. The most abundant - and most studied - SCFAs are acetate, propionate and butyrate. However, it is not clear which are the optimal dose and proportion for a beneficial effect on stress-related disorders, let alone which could be the edible ingredients capable of providing such proportions.

US6753019 B1 describes food supplements containing food fibres, which have beneficial effects for bowel health, shown by increased levels of SCFAs. US20150272982 A1 describes a nutritional composition comprising dietary fibre, a peptide having 7 or more amino acid units, a tryptophan source (which optionally could be the peptide) for use in the treatment of a negative emotion or introvert behaviour, where the fibre is linked to the release of SCFAs acetate, propionate and butyrate.

Accordingly, there is a need to identify the proportion of SCFA in the gut which is associated to a beneficial effect on modulating stress response to different life stressors.

There is also a need to identify edible ingredients, as well as food compositions comprising them, which would be capable of delivering such proportion of short chain fatty acids in the gut and thus to be used in attenuation of stress response and/or prevention of chronic stress and of conditions thereby associated. Further, there is a need to identify edible ingredients, as well as food compositions comprising them, which would be capable of delivering such proportion of short chain fatty acids in the gut and thus to be used in modulation of HPA axis reactivity leading to a decrease in cortisol output to prevent later consequences of chronic stress.

**Summary of the invention**

The present invention provides as solution to the mentioned problems as set out in the claims.

By using a mix of SCFAs in a capsule delivered directly to the colon (where the SCFAs are normally produced through fermentation of fibers by the microbiota), the inventors demonstrated a decrease in stress response (measured by decrease in cortisol response to acute stress) after 1 week of intake in healthy individuals compared to a placebo capsule. The proportion of SCFAs used was surprisingly different from that commonly found in the colon after administration of other fibers (e.g. inulin, Partially Hydrolyzed Guar Gum (PHGG) or acacia gum whereby typically the ratio acetate:propionate:butyrate is about~70:20:10). The inventors tested 2 doses of the same SCFA mix having a ratio acetate:propionate:butyrate of 72:6:22 and reflecting the SFCA ratio obtained upon fermentation of 10 or 20 g of AXOS as described in the experimental section under Example 1.

In one aspect, the present invention relates to an edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals to 100 for use in attenuation of stress response and/or prevention of chronic stress and of conditions thereby associated, wherein the edible ingredient is arabinoxylan oligosaccharide fiber (for example AXOS).

In another aspect, the present invention relates to a food composition comprising an edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 95-100% for use in attenuation of stress response and/or prevention of chronic stress and of conditions thereby associated.

## Brief description of the drawings

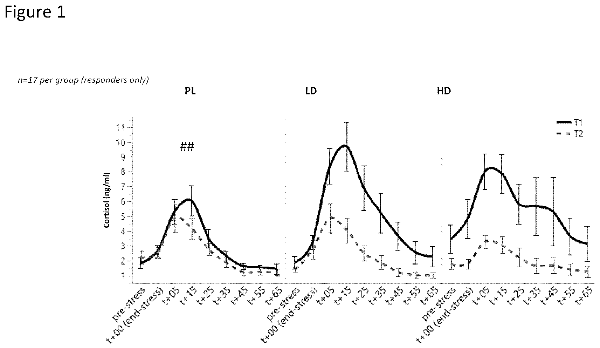

**Figure 1** is a graphical representation of results from Example 2. It shows a greater decrease in cortisol levels in the high dose (HD) of the tested fiber in comparison with the placebo group as well as in the low dose (LD) of the tested fiber in comparison with the placebo group (PL).

## Detailed description of the invention

Within the context of the present invention, the terms **"treating"** or **"treatment"** mean to decrease or alleviate the symptoms of a condition and/or illness suffered by a mammal, in particular an animal or human being.

The terms **"prevention"** or **"preventing"** mean to stop the onset of symptoms or to reduce the severity of such symptoms of a condition and/or illness suffered by a mammal, in particular an animal or human being. In addition the terms **"prevention"** or **"preventing"** mean to delay the onset of symptoms.

Within the context of the present invention, the term "attenuating stress response" includes helping subjects (healthy, anxious and/or stressed) to cope with stressors (including psychosocial stressors) in life and to manage stressful events, by decreasing their stress response to such situations. This also helps avoiding that these evolve into a chronic stress for the subject and/or the associated consequence for health.

Within the context of the present invention, the term "chronic stress" indicated a sustained situation of exposure for a subject to stressors in life (including psychosocial stressors) resulting in increased, chronic activation of the HPA (Hypotalamic-Pituitary-Adrenocortical) axis that can result at long-term in a negative feedback to the HPA axis.

Within the context of the present invention, the term "conditions associated to chronic stress" includes sleep disturbances, concentration and memory problems, chronic fatigue, and many serious health problems, for example mental illness such as, depression, anxiety, and burnout. Additionally, conditions associated to chronic stress include cardiovascular disease, including heart disease, high blood pressure, abnormal heart rhythms, heart attacks, and stroke.

Within the context of the present invention, the term "edible ingredient" means any kind of edible ingredient or mixture of ingredients that may be safely consumed by a human or animal.

Within the context of the present invention, the term "food composition" means any kind of edible composition that may be safely consumed by a human or animal. Said food composition may be in solid, semi-solid or liquid form and may consist of or comprise one or more nutrients, foods or nutritional supplements. For instance, the food composition may additional comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form). Non limiting examples of food compositions are selected from the list consisting of: dairy product, pet food product, nutritional composition, maternal composition and dietary supplements.

The term **"dairy products",** as used herein, refers to food products produced from animals such as cows, goats, sheep, yaks, horses, camels, and other mammals. Examples of dairy products are low-fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term **"pet food product"** as used herein refers to a nutritional product that is intended for consumption by pets. A pet, or companion animal, as referenced herein, is to be understood as an animal selected from dogs, cats, birds, fish, rodents such as mice, rats, and guinea pigs, rabbits, etc.

As used herein, the term **"nutritional composition"** includes, but is not limited to, complete nutritional compositions, partial or incomplete nutritional compositions, nutritional supplements, and disease or condition specific nutritional compositions.

As used herein, **"Maternal composition"** refers to a composition and/or nutritional composition for maternal administration in a mammal, in particular a woman, which may occur pre-, during, and/or post- pregnancy.

The term **"nutritional supplement"**, or **"dietary supplement",** as used herein, refers to a nutritional product that provides nutrients to an individual that may otherwise not coveniently be consumed in sufficient quantities by said individual.

Supplements can for example be provided in the form of a pill, a tablet a lozenger, a chewy capsule or tablet, a tablet or capsule, or a powder supplement that can for example be dissolved in water or sprinkled on food. Most preferred is a powder supplement that can be dissolved in liquid or sprinkled on food, most preferably dissolved in water. Such supplements typically provide the selected nutrients while not representing a significant portion of the overall nutritional needs of the subject. Typically they do not represent more than 0.1%, 1%, 5%, 10% or 20% of the daily energy need of the subject.

Within the context of the present invention, the expression **"Short chain Fatty Acid (also SCFA)"** identifies propionic acid, formic acid, acetic acid, butyric acid, valeric acid, iso-butyric acid and iso-valeric acid and/or physiologically acceptable salts thereof (such as for example calcium, potassium, sodium, magnesium, ammonium salts of propionate, formate, acetate, butyrate, valerate, iso-butyrate and iso-valerate) or mixtures thereof.

SCFAs are mainly synthesized by fermentation. In humans, SCFAs are produced in the gastrointestinal tract in the intestine; the main production site is the colon due to microbial density. SCFAs are metabolites of fermentable material in the colon and derived mainly from complex carbohydrates or fermentable fibers such as lactose, prebiotics and oligosaccharides.

Within the context of the present invention the term **"edible ingredient providing a short chain fatty acid in the gut"** indicates an edible ingredient (either an individual ingredient or a mixture of ingredients) which upon administration to a human and/or animal is able to generate SCFAs in the grastrointestinal tract of such animal and /or human fermentation, especially in the colon, as a result of the animal and/or human metabolism and/or of the metabolism of their intestinal microbiota.

Within the context of the present invention, the term **"fermentable fiber"** identify an edible ingredients (either an individual ingredient or a mixture of ingredients) which by mammal/animal/microbial metabolism give rise to SCFAs in the human/animal body of the subject assuming them, such as for example fermentable oligosaccharides and starches. The fermentable fibers can include but are not restricted to pectins, mucilages, gums, galacto-oligosaccharides, oligofructans, inulin, polyfructoses, arabinoglactans, hemicelullose, oligosaccharides or mixtures of thereof.

Within the context of the present invention, the term **"arabinoxylan"** indicates a hemicellulose found in both the primary and secondary cell walls of plants, including woods and cereal grains and consisting of copolymers of two pentose sugars: arabinose and xylose. Arabinoxylan chains contain a large number of 1,4-linked xylose units. Many xylose units are substituted with 2, 3 or 2,3-linked arabinose residues.

Within the context of the present invention, the term **"Arabinoxylan-oligosaccharide"** indicates an ingredient composed of oligosaccharides from arabinoxylan. For example, a commercially available Arabinoxylan-oligosaccharide ingredient is AXOS, sold by Purefiber, which is an ingredient composed of oligosaccharides from arabinoxylan. AXOS composition is well documented, constant and guaranteed throughout the year by Purefiber.

The invention pertains to the subject-matter of the claims.

In one embodiment of the present invention, the edible ingredient is for use in attenuation of stress response.

In another embodiment of the present invention, the edible ingredient is for use in prevention of chronic stress and of conditions thereby associated. In such embodiment, the edible ingredient is for use in modulation of HPA axis reactivity leading to a decrease in cortisol output to prevent later consequences of chronic stress.

In one embodiment, the conditions associated to chronic stress are selected in the group consisting of: sleep disturbances, concentration and memory problems, chronic fatigue, mental illness, such as depression, anxiety, or burnout, and cardiovascular disease, for example heart disease, high blood pressure, abnormal heart rhythms, heart attacks, or stroke.

In one embodiment, the edible ingredient according to the present invention provides a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 100.

In another embodiment, the edible ingredient according to the present invention provides a short chain fatty acid ratio in the gut such that acetate is in a ratio ranging from 70 to 75, propionate is in a ratio ranging from 4 to 8, butyrate is in a ratio ranging from 20 to 24 and wherein the sum of individual ratios for acetate, propionate and butyrate equals 100.

In a still further embodiment, the edible ingredient according to the present invention provides a short chain fatty acid ratio in the gut of acetate:propionate: butyrate of 72:6:22.

In one embodiment, the edible ingredient is selected in the list consisting of: AXOS (arabinoxylooligosaccharides) fiber, soluble arabinoxylan, whole grain wheat, for example flaked wholegrain wheat, a mixture of oligofructose and high amylose corn starch, a mixture of oligofructose, high amylose corn starch, and partially hydrolysed guar gum, a mixture of oligofructose, high amylose corn starch and wheat bran, and a mixture of oligofructose, high amylose corn starch, partially hydrolysed guar gum and wheat bran, for example extruded wheat bran.

In another embodiment, the edible ingredient according to the present invention is arabinoxylooligosaccharide (for example AXOS) fiber.

In one embodiment of the present invention, the food comprises at least 5g of the edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 100.

In another embodiment of the present invention, the food composition comprises at least 8 g, for example at least 10 g of the edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 100.

As it will be apparent to a person skilled in the art, fermentation of fibers in the gut may also generate amounts of additional short chain fatty acid or salts thereof such as valerate, iso-butyrate and iso-valerate. Such additional short chain fatty acids or salts thereof may be present in amounts up to 10, for example 5 % molarity of total SCFA (thus also including acetate, propionate and butyrate).

In one embodiment of the present invention, it is provided a an edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 100 for use in attenuation of stress response and/or prevention of chronic stress and of conditions thereby associated and wherein the edible ingredient also generates additional short chain fatty acids in the gut selected in the group consisting of: valerate, iso-butyrate and iso-valerate, in an amount up to 10%, for example 5% w/w of the total short chain fatty acids.

**Experimental Section**

**Example 1**

**Identification of SFCA ratio in the gut upon fermentation of AXOS fiber**

**Plasma concentrations of 13C-SCFA after 13C-AXOS and 13C-DF-B consumption in healthy subjects**

Subjects

Healthy subjects were recruited among the student population of the Group Biomedical Sciences of KU Leuven and the staff of the University Hospital Leuven. All subjects were aged between 18 and 65 years and had a BMI between 18.5 and 27 kg/m$^2$. In addition, participants had a regular diet pattern consisting of three meals per day for at least five days a week. Exclusion criteria were previous abdominal surgery (except from appendectomy), history of chronic gastro-intestinal conditions such as inflammatory bowel diseases, irritable bowel syndrome and celiac disease, or being on a low calorie or vegetarian diet. Subjects were excluded if they had donated blood during the last three months, suffered from low blood haemoglobin levels or had participated in experiments involving ionizing radiation during the last year. Female subjects were excluded if pregnant or lactating. All subjects were free of medication influencing the gut transit or intestinal microbiota for 14 days and of antibiotics for one month. Intake of pre- and probiotics was prohibited during the study period.

The study procedure was approved by the Ethics Committee of the Leuven University Hospital, Belgium. Written, informed consent was obtained from all subjects and the study conformed to the Declaration of Helsinki. The study was registered on ClinicalTrails.gov (NCT02378376).

Study design

The study consisted of two test days with minimal one week in between, in which the fermentation of either 13C-AXOS (C-labelled arabinoxylan oligosaccharides) or 13C-DF-B (C-labelled Dietary Fiber-enriched Bran) was assessed. The order of the test days was randomised using internet-based software (www.randomizer.com). On the morning of the test day, subjects came to the laboratory after an overnight fast and collected baseline breath for analysis of $^{13}CO_2$, $^{14}CO_2$ and $H_2$ and urine samples. Subsequently, a catheter was introduced in an antecubital vein of the forearm. One catheter was used to collect basal and postprandial blood samples during the test day and in the contralateral arm a continuous primed infusion with deuterated SCFA. This infusion was applied to allow calculating the plasma clearance of the SCFA.

For breakfast, the subjects received 250 g of low fat yoghurt to which either 15.7 g 13C-labelled AXOS or 22.2 g 13C labelled DF-B were added. In addition, a marker for orocecal transit time (OCTT) was added to the breakfast. After breakfast, breath and blood samples were collected during the test day up to 14 h. After 14 h, the subjects were allowed to go home for the night but returned to the laboratory on the next morning for an additional blood sample 24 h after fibre consumption.

Analytical procedures

Analysis of SCFA in plasma

Total (labelled + unlabelled) SCFA concentrations in plasma were measured using GC.

$^{13}$C- and $^2$H-enrichments of SCFA in plasma and urine

The $^{13}C/^{12}C$ ratios of SCFA were measured using GC combustion isotope ratio mass spectrometry. To calculate the enrichment of the SCFA from the measured $^{13}CO_2$ enrichment, the number of labelled carbon atoms per molecule and the enrichment of the administered substrate ($^{13}$C-AXOS and $^{13}$C-DF-B) were taken into account. Plasma concentrations of $^{13}$C SCFA originating from the colon at each time point ($n_{colon}$) were calculated according:

$$n_{colon} = n_{(t)} \times (\llbracket AP \rrbracket_t - \llbracket AP \rrbracket_{(t_0)})/(\llbracket AP \rrbracket_{colon} - \llbracket AP \rrbracket_{(t_0)})$$

(Equation 1)

with nt: total concentration of SCFA in plasma at time point t (labelled + unlabelled), APt: $^{13}$C enrichment of the SCFA measured in plasma at time point t, APt0: $^{13}$C enrichment of the SCFA measured in plasma at time 0 and APcolon: $^{13}$C enrichment of the administered substrate. We assumed that the $^{13}$C-SCFA produced in the colon by bacterial fermentation had the same enrichment as the substrates $^{13}$C-AXOS and $^{13}$C-DF-B.

The $^2$H-enrichments of SCFA were measured using GC mass spectrometry. The $^2$H-SCFA plasma concentrations at each time point were calculated by multiplying total SCFA concentrations in plasma with the $^2$H-enrichment of the SCFA.

SCFA clearance was determined in each subject from the applied constant infusion of $^2$H-SCFA and the measured plasma concentrations of $^2$H-SCFA according to:

$$\llbracket Cl \rrbracket_{SCFA} = I/Css$$

(Equation 2)

With: I as the $^2$H-SCFA infusion rate (nmol x h-1), and Css as the $^2$H-SCFA steady state concentration in plasma ($\mu$mol x L-1).

$^{13}$C SCFA plasma concentrations versus time graphs were constructed and the cumulative amount (AUC) of each $^{13}$C SCFA in plasma was calculated. The total amounts of SCFA that appeared in the systemic circulation over a period of 12 h were calculated using the AUC of the SCFA concentrations-time curve and the SCFA clearance of each subject according to:

$$SCFA \text{ in plasma after } 12 \text{ h} = \llbracket AUC \rrbracket_{SCFA} \times \llbracket Cl \rrbracket_{SCFA}$$

(Equation 3)

with: AUC as the area under the SCFA concentration-time curve ($\mu$mol x h x L-1), and Cl as clearance of SCFA from the plasma (L x h-1).

Subsequently, the AUC of each SCFA were converted to their production in the colon using systemic availability values of 36%, 9% and 2% for acetate, propionate and butyrate respectively.

Table 1 below reports the results of this experiment: amount of SCFA in colon (mmol) and corresponding SFCA proportion for each subject and mean thereof.

Table 1

| Subject | 13C-Acetate | 13C-Propionate | 13C-Butyrate | Acetate | Propionate | Butyrate |
|---------|-------------|----------------|--------------|---------|------------|----------|
| 1 | 158,63 | 12,15 | 25,66 | 80,8 | 6,2 | 13,1 |
| 2 | 96,22 | 1,90 | 121,94 | 43,7 | 0,9 | 55,4 |
| 3 | 163,30 | 26,82 | 24,74 | 76,0 | 12,5 | 11,5 |
| 4 | 248,44 | 4,36 | 34,91 | 86,4 | 1,5 | 12,1 |
| 5 | 216,70 | 9,35 | 162,16 | 55,8 | 2,4 | 41,8 |
| 6 | 270,29 | 20,43 | 31,82 | 83,8 | 6,3 | 9,9 |
| 7 | 215,14 | 4,53 | 58,57 | 77,3 | 1,6 | 21,0 |
| 8 | 432,58 | 10,94 | 83,86 | 82,0 | 2,1 | 15,9 |
| 9 | 197,28 | 6,81 | 116,24 | 61,6 | 2,1 | 36,3 |
| 10 | 189,80 | 33,18 | 13,76 | 80,2 | 14,0 | 5,8 |
| | | | | | | |
| **Mean** | | | | 72,8 | 5,0 | 22,3 |

**Example 2**

**Clinical Trial for response to acute stress upon administration of capsules releasing SFCA in the gut in a ratio of Acetate: Propionate: Butyrate being 72:6:22**

**Materials and Methods**

**a) Subjects**

A power calculation was done based on data available from the stress-induction protocol (MAST; Smeets et al., 2012). Power was calculated using the General Linear Multivariate Model Power & Sample Size software (GLIMMPSE, http://glimmpse.samplesizeshop.org/#/). Assuming a 10% and 20% reduction in stress response for the low- and high-dose SCFA groups, respectively (no previous data available to estimate the effect), a total sample size of 66 participants (n = 22 per treatment arm) yielded sufficient power (84%).

Sixty-seven dutch-speaking male participants with a mean age of 23.6 (SD=2.4) and normal BMI (M=22.5 SD=1.9) were recruited for the clinical trial. Exclusion criteria included having previous or current neurological, psychiatric, gastrointestinal or endocrine disorders, substance/alcohol dependence or abuse (> 2 units per day/14 units per week), current or recent regular medication use, one or more diagnoses based on the mini-international neuropsychiatric interview, one or more diagnoses based on ROME IV for GI disorders, night-shift work, adherence to vegan or vegetarian diets, smoking, using pre- or probiotics within one month preceding the study, using antibiotics within three months preceding the study, or having previous experience with one of the psychological tasks employed in the study. One participant was excluded from data analysis due to non-compliance. This clinical trial was approved by the Medical Ethics Committee of UZ Leuven/KU Leuven (S60501) and *was conducted* in accordance with the Declaration of *Helsinki*. ClinicalTrials.gov Identifier: NCT03688854.

**b) Experimental protocol**

We conducted a one-week, randomized, triple-blind, placebo controlled trial performed according to a parallel group design, where one group received a low SCFA dose equivalent to 10 grams of AXOS, another group received a high SCFA dose equivalent to 20 grams of AXOS, and a third group received placebo (cellulose).

The clinical trial comprised two study visits. Visit 1 was a baseline visit, prior to the commencement of intervention, and visit 2 was scheduled one week later, following the SCFA or placebo intervention. Participants adhered to a low-fiber diet starting three days prior to visit 1 up until the end of the clinical trial (i.e., 11 days, instructions can be found in Supplementary Material). The purpose of the low-fiber diet to standardize fiber intake across the three groups.

Participants kept food diaries for the three consecutive days preceding each study visit via www.fatsecret.com. Each study visit commenced with the collection of saliva samples upon waking for one hour to quantify CAR. This was followed by the consumption of a no-fiber breakfast at home on study visit 1, and consuming the same breakfast with the 32 capsules on the morning of study visit 2. Participants arrived to the laboratory three hours later and consumed a standardized no-fiber lunch *ad libitum*. Participants then performed the Maastricht Acute Stress Test (MAST) and collected saliva samples for 65 minutes following the termination of the stressor.

**c) Colon delivery capsules of SCFA and placebo**

To mimic the fiber fermentation process, SCFAs were delivered directly to the colon using pH-dependent colon delivery capsules (CDC). Cellulose was used as placebo in accordance with Daud et al.[30]. Participants in the high dose SCFA group consumed 32 capsules of SCFA mixture (174.2 mmol acetate, 13.3 mmol propionate, and 52.4 mmol butyrate) daily. Participants in the low dose SCFA group consumed 16 capsules of SCFA mixture 87.1 mmol acetate, 6.6 mmol propionate, and 26.2 mmol butyrate) as well as 16 placebo capsules daily. The placebo group consumed 32 placebo capsules (cellulose) daily. To promote compliance, participants were allowed to consume the capsules throughout the day and were asked to bring back the non-consumed capsules.

**d) Psychological readouts**

Stress sensitivity

The Maastricht Acute Stress Test (MAST), devised by Smeets et al. (2012), was employed in this clinical trial to assess participant's responses to acute psychosocial stress at the level of HPA-axis activity (salivary cortisol) as well as subjective stress (ratings). The MAST induces stress at the physical, cognitive, and social level. The task consists of a 5-min preparation phase and a 10-min acute stress phase. The 5-min preparation period serves to seat participants in front of a computer screen and instruct them about the upcoming task in which they have to alternate between placing their hand in cold water and performing mental arithmetic, all while being videotaped so as to later analyze their facial expressions and being monitored by the experimenter. The task consisted of multiple trials in which participants had to immerse their hand in ice-cold (2 °C) water for a period that does not exceed 90 s. In between the hand immersion trials, participants engaged in the mental arithmetic test, which consisted of counting backwards starting at 2043 in steps of 17 or 13 (counterbalanced across the groups and study visits) as quickly and as accurately as possible. Participants were given negative feedback upon making a mistake and were never provided with positive feedback. They were required to continue with the mental arithmetic until the computer would signal the start of the next hand immersion trial, which would take at least 45 s. In reality, the duration of the various hand immersion trials and the mental arithmetics were fixed for all participants. This task has been validated and serves to elicit a robust subjective and physiological stress response. Moreover, no significant habituation or sensitization to the MAST was found following repeated administration, which is crucial to the present study.

*Salivary cortisol*

Saliva samples were obtained with synthetic Salivette 5 min before (i.e., $t_{\text{pre-stress (-20 min)}}$) and 8 times after stress induction (i.e., $t_{+00}$, $t_{+05}$, $t_{+15}$, $t_{+25}$, $t_{+35}$, $t_{+45}$, $t_{+55}$ and $t_{+65}$ min with reference to the end of the stressor). Samples were stored at -20 °C until analysis.

*Visual analogue scale*

Prior to the MAST, in the middle, and at the end, participants were asked to rate how stressful, how painful, and how unpleasant the stress induction procedure had been for them on a visual analogue scale (VAS; anchors: 0 = *"not at all"*; 10 = *"extremely"*). This was used to measure the subjective stress response.

Cortisol analysis

Saliva samples collected for the analysis of CAR and HPA-axis response to MAST and stored at -20°C until analysis. The samples were analysed using Salivary Cortisol ELISA SLV-2930 (DRG Instruments GmbH, Margburg, Germany) according to manufacturer's instruction. The range of the assay is between 0.09 - 30 ng/mL.

**Results**

**Colonic delivery of SCFAs attenuates the cortisol response to acute psychosocial stress**

The Maastricht Acute Stress Task (MAST) was administered prior to and after one week of colonic delivery of SCFA in order to evaluate changes in participants' stress response to a combination of acute physical, cognitive, and social stressors. Only participants who responded to the MAST prior to SCFA administration, as indicated by an increase in cortisol response from baseline-to-peak by 15.5%, were included in the analysis. This led to a sample of 17 participants in each treatment group. Linear mixed models revealed that the interventions had different effects on cortisol response to acute stress (significant group x visit interaction effect ($F(2,48) = 4.76$, $p = 0.01$, but no three-way group x visit x sample-number interaction effect ($F(16,279) = 0.74$, $p = 0.76$)). Follow-up planned contrasts revealed that there was a greater decrease in cortisol levels in the high dose (HD) in comparison with the placebo group (PL) ($t(48) = 2.63$, $p = 0.035$), as well as in the low dose (LD) in comparison with the placebo group (PL) ($t(48) = -2.58$, $p = 0.035$) (see Figure 1). There was no dose-effect relationship, as indicated by a lack of a significant difference between the high and low dose groups ($t(48) = 0.36$, $p = .72$).

**Example 3**

**Identification of alternative ingredients providing a short chain fatty acid ratio in the gut as claimed and Protocol for batch fermentation**

A skilled person is able to determine appropriate selection of fiber to achieve the claimed short chain fatty acid ratio, by selecting fibers known for high acetate and butyrate, and low propionate production during in vitro fermentation.

The skilled person would then be able to confirm the selection of fiber by testing the ingredient in a protocol of batch fermentation as below described.

Among fibers suitable for high acetate and butyrate production during fermentation we can cite: Oligo-fructose, Fructo-oligosaccharide, Whole grain wheat, Inulin, Chicory Root fiber or some Pectin. Among fibers suitable for high butyrate production during fermentation we can cite for example: resistant starch, high amylose wheat.

Batch fermentation may be performed in vessels containing bacterial a liquid nutritional media adapted to support the growth of colonic bacteria. All test ingredients (fibers) may be added to sugar-depleted nutritional medium containing basal nutrients that are present in the colon (e.g. host-derived glycans such as mucin) together with a freshly prepared human faecal inoculum. Faecal inocula are obtained from healthy adult donors.

Incubations are performed during 48 h, at 37°C, under shaking (90 rpm) and anaerobic conditions. A blank is also included, containing only sugar-depleted nutritional medium (without fibers) to assess the background activity of the community. This procedure allows to assess the specific effect of test ingredients on the metabolic and community composition profile of the colonic microbiota.

The microbiota profile is obtained by 16S-targeted Illumina sequencing on collected samples. The SCFAs were measured using a solid phase microextraction technic (SPME) coupled to gas chromatography mass spectrometry (GC/MS) method. The GC/MS allow to perform quantification of the various SCFAs via an external linear calibration curve using labelled internal standard. The quantity of each SCFAs is express in mmol produced during the 48h of fermentation. Thus, we are able to calculate the proportion of each produced during the fermentation of fiber.

### Example 4

**Description of alternative ingredients delivering in the gut in a ratio of Acetate: Propionate: Butyrate being 72:6:22**

By way of further examples, the following ingredients based on single fibers or mixture of fibers can alternatively be used according to the present invention for the treatment and/or prevention of stress-related disorders. Table 2 reports examples of alternative ingredients providing a ratio of Acetate:Propionate:Butyrate = 72:6:22.

**Table 2**

| | | | | |
|---|---|---|---|---|
| **Nb of fibers in the blend** | N=1 | 15.7g Whole grain wheat, flaked | 31.5g Whole grain wheat, flaked | 62.9g Whole grain wheat, flaked |
| | N=2 | 4.9g Oligofructose & 4.3g High Amylose corn starch,RS | 9.8g Oligofructose & 8.5g High Amylose corn starch, RS | 19.5g Oligofructose & 17.1g High Amylose corn starch, RS |
| | N=3 | 4.9g Oligofructose & 3.9g High Amylose corn starch,RS & 0.7g partially hydrolyzed guar gum | 9.4gOligofructose & 6.5g High Amylose corn starch, RS& 4 Wheat Bran | 18.9g Oligofructose& 13g High Amylose corn starch, RS & 7.9 Wheat Bran |
| | N=4 | 4.8g Oligofructose & 3.9g High Amylose corn starch,RS & 0.5g Partially hydrolyzed guar gum & 0.5g wheat bran, extruded | 9.7g Oligofructose & 7.8g High Amylose corn starch, RS & 1.2 Partially Hydrolyzed guar gum & 0.5g wheat bran extruded | 19.5g Oligofructose& 15.7g High Amylose corn starch, RS & 2.5g partially hydrolyzed guar gum & 0.5 wheat bran extruded |

### Claims

1. Edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 100 for use in attenuation of stress response and/or prevention of chronic stress and of conditions thereby associated, wherein the edible ingredient is arabinoxylan oligosaccharide fiber (for example AXOS).

2. Edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 100 for use in attenuation of stress response and/or prevention of chronic stress and of conditions thereby associated, wherein the edible ingredient is selected in the list consisting of: 15.7g flaked Whole grain wheat, 31.5g flaked Whole grain wheat, 62.9g flaked Whole grain wheat, 4.9g Oligofructose and 4.3g High Amylose corn starch,RS, 9.8g Oligofructose and 8.5g High Amylose corn starch, RS, 19.5g Oligofructose and 17.1g High Amylose corn starch, RS, 4.9g Oligofructose, 3.9g High Amylose corn starch, RS and 0.7g partially hydrolyzed guar gum, 9.4g Oligofructose, 6.5g High Amylose corn starch, RS and 4g Wheat Bran, 18.9g Oligofructose, 13g High Amylose corn starch, RS and 7.9 Wheat Bran, 4.8g Oligofructose, 3.9g High Amylose corn starch,RS, 0.5g Partially hydrolyzed guar gum and 0.5g wheat bran, extruded, 9.7g Oligofructose, 7.8g High Amylose corn starch, RS, 1.2 Partially Hydrolyzed guar gum and 0.5g wheat bran extruded, and 19.5g Oligofructose, 15.7g High Amylose corn starch, RS, 2.5g partially hydrolyzed guar gum and 0.5 wheat bran extruded.

**3.** Edible ingredient for use according to any of the preceding claims in attenuation of stress response and/or prevention of chronic stress and of conditions thereby associated selected in the group consisting of: sleep disturbances, concentration and memory problems, chronic fatigue, mental illnesses and cardiovascular diseases.

**4.** Edible ingredient for use according to any of the preceding claims in decreasing stress response to stressors in life and/or stressful events.

**5.** Edible ingredient for use according to any of the preceding claims, providing a short fatty acid ratio in the gut of acetate: propionate: butyrate being 72:6:22.

**6.** Food composition comprising an edible ingredient providing a short chain fatty acid ratio in the gut such that acetate is in a ratio ranging from 70 to 80, propionate is in a ratio ranging from 2 to 8, butyrate is in a ratio ranging from 20 to 25 and wherein the sum of individual ratios for acetate, propionate and butyrate equals 100 as described in any of the preceding claims for use in attenuation of stress response and/or prevention of chronic stress and of conditions thereby associated, for example selected in the group consisting of: sleep disturbances, concentration and memory problems, chronic fatigue, mental illnesses and cardiovascular diseases.

**Patentansprüche**

**1.** Essbare Zutat, die im Darm ein Verhältnis kurzkettiger Fettsäuren bereitstellt, sodass Acetat in einem Verhältnis von 70 bis 80, Propionat in einem Verhältnis von 2 bis 8 und Butyrat in einem Verhältnis von 20 bis 25 vorliegt und wobei die Summe der Einzelverhältnisse für Acetat, Propionat und Butyrat 100 ergibt, zur Verwendung bei der Abschwächung von Stressreaktionen und/oder der Vorbeugung von chronischem Stress und damit verbundenen Zuständen, wobei die essbare Zutat Arabinoxylan-Oligosaccharid-Faser (beispielsweise AXOS) ist.

**2.** Essbare Zutat, die ein Verhältnis kurzkettiger Fettsäuren im Darm bereitstellt, sodass Acetat in einem Verhältnis von 70 bis 80, Propionat in einem Verhältnis von 2 bis 8 und Butyrat in einem Verhältnis von 20 bis 25 vorliegt und wobei die Summe der einzelnen Verhältnisse für Acetat, Propionat und Butyrat 100 ergibt, zur Verwendung bei der Abschwächung von Stressreaktionen und/oder der Vorbeugung von chronischem Stress und damit verbundenen Zuständen, wobei die essbare Zutat ausgewählt ist aus der Liste bestehend aus: 15,7 g Vollkornweizenflocken, 31,5 g Vollkornweizenflocken, 62,9 g Vollkornweizenflocken, 4,9 g Oligofructose und 4,3 g Maisstärke mit hohem Amylosegehalt, RS, 9,8 g Oligofructose und 8,5 g Maisstärke mit hohem Amylosegehalt, RS, 19,5 g Oligofructose und 17,1 g Maisstärke mit hohem Amylosegehalt, RS, 4,9 g Oligofructose, 3,9 g Maisstärke mit hohem Amylosegehalt, RS und 0,7 g teilweise hydrolysiertes Guarkernmehl, 9,4 g Oligofructose, 6,5 g Maisstärke mit hohem Amylosegehalt, RS und 4 g Weizenkleie, 18,9 g Oligofructose, 13 g Maisstärke mit hohem Amylosegehalt, RS und 7,9 Weizenkleie, 4,8 g Oligofructose, 3,9 g Maisstärke mit hohem Amylosegehalt, RS, 0,5 g teilweise hydrolysiertes Guarkernmehl und 0,5 g Weizenkleie, extrudiert, 9,7 g Oligofructose, 7,8 g Maisstärke mit hohem Amylosegehalt, RS, 1,2 teilweise hydrolysiertes Guarkernmehl und 0,5 g Weizenkleie extrudiert, und 19,5 g Oligofructose, 15.7 g Maisstärke mit hohem Amylosegehalt, RS, 2,5 g teilweise hydrolysiertes Guarkernmehl und 0,5 extrudierte Weizenkleie.

**3.** Essbare Zutat zur Verwendung nach einem der vorstehenden Ansprüche zur Abschwächung von Stressreaktionen und/oder zur Vorbeugung von chronischem Stress und damit verbundenen Zuständen, ausgewählt aus der Gruppe bestehend aus: Schlafstörungen, Konzentrations- und Gedächtnisproblemen, chronischer Müdigkeit, psychischen Erkrankungen und Herz-Kreislauf-Erkrankungen.

**4.** Essbare Zutat zur Verwendung nach einem der vorstehenden Ansprüche zur Verringerung der Stressreaktion auf Stressfaktoren im Leben und/oder stressige Ereignisse.

**5.** Essbare Zutat zur Verwendung nach einem der vorstehenden Ansprüche, die im Darm für ein niedriges Fettsäureverhältnis von Acetat: Propionat: Butyrat von 72:6:22 sorgt.

**6.** Nahrungsmittelzusammensetzung, die eine essbare Zutat umfasst, die im Darm ein Verhältnis kurzkettiger Fettsäuren bereitstellt, sodass Acetat in einem Verhältnis von 70 bis 80, Propionat in einem Verhältnis von 2 bis 8 und Butyrat in einem Verhältnis von 20 bis 25 vorliegt und wobei die Summe der einzelnen Verhältnisse für Acetat, Propionat und Butyrat 100 ergibt, wie in einem der vorstehenden Ansprüche beschrieben, zur Verwendung bei der Abschwächung von Stressreaktionen und/oder der Vorbeugung von chronischem Stress und damit verbundenen Zuständen, beispielsweise ausgewählt aus der Gruppe bestehend aus: Schlafstörungen, Konzentrations- und Gedächtnisproblemen, chronischer Müdigkeit, psychischen Erkrankungen und Herz-Kreislauf-Erkrankungen.

**Revendications**

1.  Ingrédient comestible fournissant un rapport d'acides gras à chaîne courte dans l'intestin tel que l'acétate est dans un rapport allant de 70 à 80, le propionate est dans un rapport allant de 2 à 8, le butyrate est dans un rapport allant de 20 à 25 et où la somme des rapports individuels pour l'acétate, le propionate et le butyrate est égale à 100, destiné à être utilisé dans l'atténuation de la réponse au stress et/ou la prévention du stress chronique et d'états qui y sont associés, où l'ingrédient comestible est une fibre d'oligosaccharide d'arabinoxylane (par exemple AXOS).

2.  Ingrédient comestible fournissant un rapport d'acides gras à chaîne courte dans l'intestin tel que l'acétate est dans un rapport allant de 70 à 80, le propionate est dans un rapport allant de 2 à 8, le butyrate est dans un rapport allant de 20 à 25 et où la somme des rapports individuels pour l'acétate, le propionate et le butyrate est égale à 100, destiné à être utilisé dans l'atténuation de la réponse au stress et/ou la prévention du stress chronique et d'états qui y sont associés, où l'ingrédient comestible est choisi dans la liste constituée de : 15,7 g de blé complet en flocons, 31,5 g de blé complet en flocons, 62,9 g de blé complet en flocons, 4,9 g d'oligofructose et 4,3 g d'amidon de maïs à haute teneur en amylose, RS, 9,8 g d'oligofructose et 8,5 g d'amidon de maïs à haute teneur en amylose, RS, 19,5 g d'oligofructose et 17,1 g d'amidon de maïs à haute teneur en amylose, RS, 4,9 g d'oligofructose, 3,9 g d'amidon de maïs à haute teneur en amylose, RS et 0,7 g de gomme de guar partiellement hydrolysée, 9,4 g d'oligofructose, 6,5 g d'amidon de maïs à haute teneur en amylose, RS et 4 g de son de blé, 18,9 g d'oligofructose, 13 g d'amidon de maïs à haute teneur en amylose, RS et 7,9 g de son de blé, 4,8 g d'oligofructose, 3,9 g d'amidon de maïs à haute teneur en amylose, RS, 0,5 g de gomme de guar partiellement hydrolysée et 0,5 g de son de blé extrudé, 9,7 g d'oligofructose, 7,8 g d'amidon de maïs à haute teneur en amylose, RS, 1,2 gomme de guar partiellement hydrolysée et 0,5 g de son de blé extrudé, et 19,5 g d'oligofructose, 15,7 g d'amidon de maïs à haute teneur en amylose, RS, 2,5 g de gomme de guar partiellement hydrolysée et 0,5 g de son de blé extrudé.

3.  Ingrédient comestible destiné à être utilisé selon l'une quelconque des revendications précédentes dans l'atténuation de la réponse au stress et/ou la prévention du stress chronique et d'états qui y sont associés choisis dans le groupe constitué de : troubles du sommeil, problèmes de concentration et de mémoire, fatigue chronique, maladies mentales et maladies cardio-vasculaires.

4.  Ingrédient comestible destiné à être utilisé selon l'une quelconque des revendications précédentes dans la diminution de la réponse au stress face à des facteurs de stress de la vie et/ou à des événements stressants.

5.  Ingrédient comestible destiné à être utilisé selon l'une quelconque des revendications précédentes, fournissant un rapport d'acides gras courts dans l'intestin d'acétate:propionate:butyrate de 72:6:22.

6.  Composition alimentaire comprenant un ingrédient comestible fournissant un rapport d'acides gras à chaîne courte dans l'intestin tel que l'acétate est dans un rapport allant de 70 à 80, le propionate est dans un rapport allant de 2 à 8, le butyrate est dans un rapport allant de 20 à 25 et où la somme des rapports individuels pour l'acétate, le propionate et le butyrate est égale à 100 comme décrit dans l'une quelconque des revendications précédentes, destinée à être utilisée pour l'atténuation de la réponse au stress et/ou la prévention du stress chronique et d'états qui y sont associés, par exemple choisis dans le groupe constitué de : troubles du sommeil, problèmes de concentration et de mémoire, fatigue chronique, maladies mentales et maladies cardiovasculaires.

Figure 1

EP 4 064 860 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6753019 B1

- US 20150272982 A1

**Non-patent literature cited in the description**

- **SMEETS et al.** Power was calculated using the General Linear Multivariate Model Power & Sample Size software. *GLIMMPSE*, 2012, http://glimmpse. samplesizeshop.org/#/